# EUROPÄISCHE PATENTSCHRIFT

(11) **EP 0 452 719 B1**
(45) Veröffentlichungstag und Bekanntmachung des Hinweises auf die Patenterteilung: **28.06.1995**
(21) Anmeldenummer: 91104907.0
(22) Anmeldetag: 27.03.1991
(51) Int. Cl.: C07C 43/225, C09K 19/30, C09K 19/12, C07C 25/18, C07C 25/24

(54) **Halogenierte Biphenyle und flüssigkristallines Medium**
Halogenated biphenyles and liquid crystal medium
Biphényles halogénés et milieu cristal liquide

(30) Priorität: 28.03.1990 DE 4009908
(43) Veröffentlichungstag der Anmeldung: 23.10.1991
(73) Patentinhaber: MERCK PATENT GmbH, D-64271 Darmstadt (DE)
(72) Erfinder: Reiffenrath, Volker, D-6108 Rossdorf (DE); Hittich, Reinhard, Dr., D-6101 Modautal (DE); Rieger, Bernhard, Dr., App.B 511 WACORE-, Yokohama-shi, Kanagawa pref. 227 (JP)

(56) Entgegenhaltungen:
- EP-A- 0 325 796
- EP-A- 0 387 032
- WO-A-87/04426
- DE-A- 4 027 840
- MOLECULAR CRYSTALS & LIQUID CRYSTALS, Band 157, April 1988, Seiten 25-41, Gordon and Breach Science Publishers S.A., Montreux, CH; J.J. MALLON et al.: "Thermotropic hydrocarbon liquid crystalline monomers and model compounds"

## Beschreibung

Die Erfindung betrifft neue halogenierte Biphenyle der Formel I 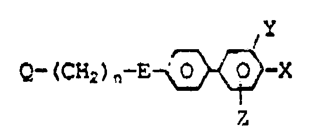
worin
- Q: Vinyl, CHF=CH-, CF₂=CH-, Cyclopropyl, CH₂Hal, CHHal₂ oder CHal₃,
- n: 1 bis 7,
- E: -O- oder eine Einfachbindung,
- X: F, Cl, -CF₃, -CN, -OCF₃ oder -OCHF₂ und
- Y und Z: jeweils unabhängig voneinander H oder F bedeuten.

Aus der JP 57-108 056-A sind Flüssigkristalle der Formel 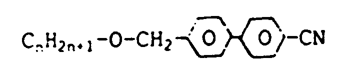
bekannt (n = 1 bis 9).

Derartige Verbindungen werden jedoch nicht den hohen Anforderungen an den elektrischen Widerstand gerecht, wie sie beispielsweise für Anzeigen mit aktiver Matrix gefordert werden. Darüberhinaus zeigen solche Verbindungen eine recht hohe Temperaturabhängigkeit der Schwellenspannung und für viele Anwendungen zu niedrige Klärpunkte bzw. ungünstige elastische Eigenschaften.

Aus der WO 87/04426 sind Flüssigkristalle, z.B. der Formel 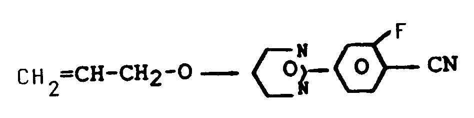
bekannt, die jedoch ungünstige elastische Eigenschaften haben und zu STN-Anzeigen mit flachen Kennlinien führen.

Die Verbindungen der Formel I können wie ähnliche, z.B. aus der DE-OS 23 56 085 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, zu hohe Temperaturabhängigkeit der Schwellenspannung.

Insbesondere bei Anzeigen vom Supertwisttyp (STN) mit Verdrillungswinkeln von deutlich mehr als 220 ° oder bei Anzeigen mit aktiver Matrix weisen die bisher eingesetzten Materialien Nachteile auf.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hervorragender Nematogenität bis zu tiefen Temperaturen, hervorragender chemischer Stabilität, ausgeprägtem ε_{⊥} bei positiver dieelektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und hohe positive dielektrische Anisotropie bei gleichzeitig günstiger Viskosität.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem Gehalt an mindestens einer Verbindung der Formel I und elektrooptische Anzeigen, die derartige Medien enthalten.

Vor- und nachstehend haben n, Q, E, X, Y und Z die angegebene Bedeutung, sofern nicht ausdrücklich etwas anderes vermerkt ist.

In den Verbindungen der Formel I sind die Alkylengruppen -(CH₂)ₙ geradkettig. Dementsprechend bedeutet CₙH₂ₙ vorzugsweise Ethylen, n-Propylen, n-Butylen, n-Pentylen, n-Hexylen oder n-Heptylen. n ist vorzugsweise 1, 2, 3 oder 4.

Der Rest 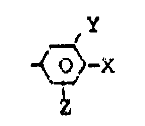
ist vorzugsweise 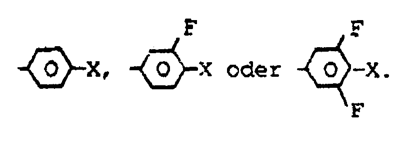
X ist vorzugsweise F, Cl, -CF₃, -OCHF₂ oder -OCF₃.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Zur Synthese der erfindungsgemäßen Verbindungen geeignete Vorstufen sind beispielsweise nach folgendem Syntheseschema erhältlich:

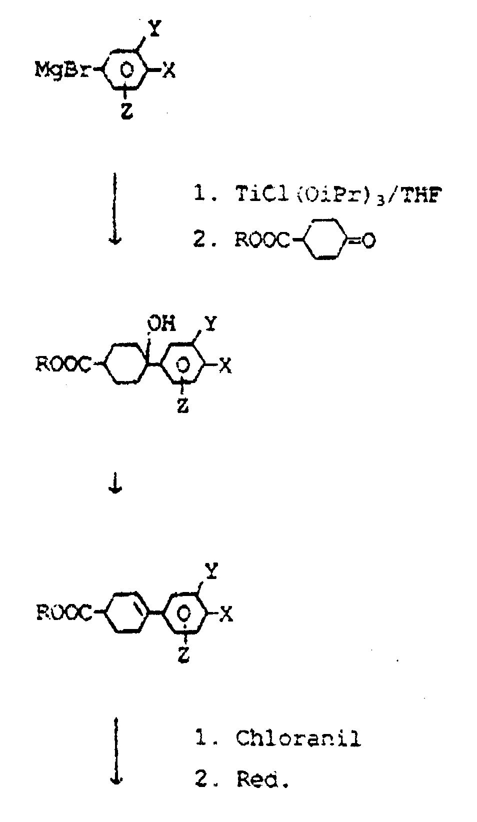

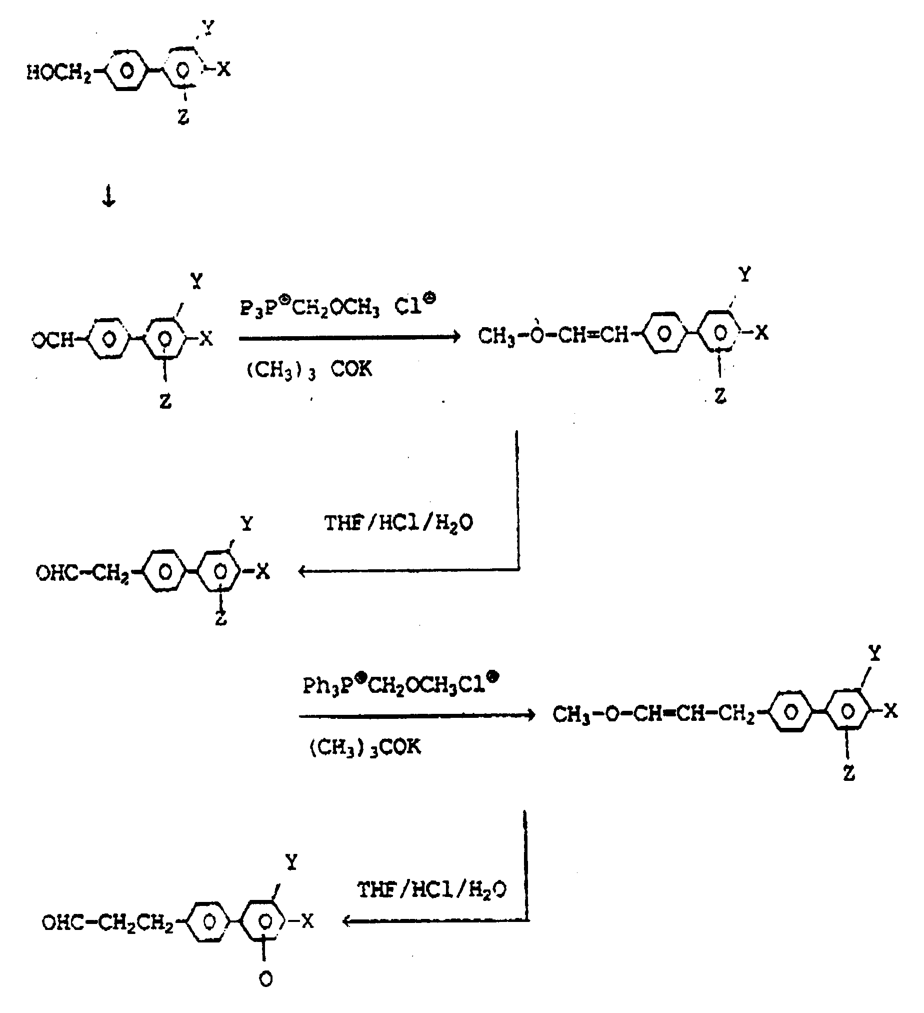

Durch sinngemäße Wiederholung dieser Reaktionssequenz sind die höheren Aldehyde erhältlich.

Aus den o.a. Aldehyden bzw. deren höheren Homologen erhält man die Verbindungen mit Q = CF₂=CH- durch Umsetzung mit Natriumchlordifluoracetat und Triphenylphosphin nach Wittig gemäß EP 0 330 216. Durch Reduktion dieser Verbindungen mit Vitride erhält man die erfindungsgemäßen Verbindungen mit Q = CHF = CH-. Die erfindungsgemäßen Vinyl- bzw. Cyclopropyl-Verbindungen erhält man ebenfalls nach Wittig durch Umsetzung mit Methyl- bzw. Cyclopropylmethyl-phosphoniumsalzen und ggf. nachfolgende Hydrierung.

Die erfindungsgemäßen Verbindungen mit Q = CH₂Hal, CHHal₂ oder CHal₃ (worin Hal F oder Cl, vorzugsweise F, bedeutet) erhält man ebenfalls nach Wittig durch Umsetzung der aus den homologen Aldehyden gemäß obigem Schema zugänglichen Phosphoniumsalzen der Formel 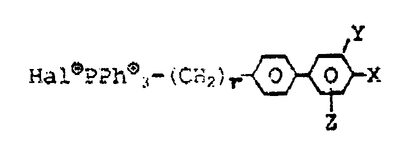
mit Aldehylden der Formel

Q-(CH₂)ₛ-CHO

(worin Q CH₂Hal, CHHal₂ oder CHal₃ bedeutet und r und s jeweils 1 bis 6 bedeuten, worin r + s = n-1) und nachfolgender Hydrierung.

Die aus dem entsprechenden Brombenzol-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Dehydrierung und Reduktion zum Benzylalkohol erhält man nach üblichen Veretherungs-Methoden die erfindungsgemäßen Verbindungen.

Die als Ausgangsstoffe verwendete Brombenzolderivate sind zum Teil bekannt, zum Teil können sie ohne Schwierigkeiten nach Standardverfahren der organischen Chemie aus literaturbedingten Verbindungen hergestellt werden. Beispielsweise sind die OCF₃- oder OCHF₂-Verbindungen nach bekannten Verfahren aus den entsprechenden Phenolen bzw. die CF₃- oder CN-Verbindungen aus den entsprechenden Benzoesäuren erhältlich. Verbindungen der Formel 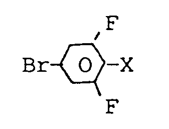
oder auch entsprechende monofluorierte Verbindungen sind beispielsweise aus den bekannten Vorstufen, in denen statt X H vorhanden ist, durch Lithiierung bei tiefen Temperaturen und anschließende Umsetzung mit einem geeigneten Elektrophil erhältlich.

Die erfindungsgemäßen Biphenylether (E = -O-) können durch übliche Veretherungsmethoden aus Vorstufen hergestellt werden, die bekannt sind oder in Analogie zu bekannten Vorstufen hergestellt werden können. Die entsprechenden Biphenylole sind durch Ether-Spaltung entsprechender Methylether erhältlich, die ihrerseits in an sich bekannter Weise durch edelmetallkatalysierte Kreuzkopplungsreaktionen hergestellt werden können (E. Poetsch, Kontakte (Darmstadt) 1988 (2) p. 15 ff.).

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl-oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)-ethane, 1-Cyclohexyl-2-biphenylylethane, 1-Phenyl-2-cyclohexylphenylethane und Tolane.

Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R'' 1

R'-L-COO-E-R'' 2

R'-L-OOC-E-R'' 3

R'-L-CH₂CH₂-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b bedeutet R'' -CN, -CF₃, -OCF₃, F, Cl oder -NCS; R hat dabei die bei den Verbindungen der Teilformeln 1a bis 5a angegebene Bedeutung und ist vorzugsweise Alkyl oder Alkenyl. Besonders bevorzugt ist R'' ausgewählt aus der Gruppe bestehend aus -F, Cl, CF₃ und -OCF₃. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeutet ferner:
- THF: Tetrahydrofuran

### Beispiel 1

Ein Gemisch aus 13,5 g p-(2-fluorethoxy)-jodbenzol, 10,9 g 3,4,5-Trifluorphenylboronsäure, 58 ml Toluol, 23 ml Ethanol 29 ml Na₂CO₃-Lsg. (2 mol/l) und 0,8 g Palladium-Kat. wird 6 h am Rückfluß gekocht. Nach üblicher Aufarbeitung und Aufreinigung durch Chromatographie und Kristellisation erhält man 3,4,5-Trifluor-4'-(2-fluorethoxy)-biphenyl, m.p. 66 °C.
Analog werden hergestellt (X = Y = Z = F, E = -O-):

| Q | n |
|---|---|
| CH₂F | 2 |
| CH₂F | 3 |
| CH₂F | 4 |
| CH₂F | 5 |
| CF₂=CH- | 2 |
| CF₂=CH- | 3 |
| CF₂=CH- | 4 |
| CHF=CH- | 2 |
| CHF=CH- | 3 |
| CHF=CH- | 4 |

### Beispiel 2

Zu einem Gemisch von 4,6 g 3,4-Difluorphenylphenol, 1,6 g 2-Fluorethanol, 6,6 g Triphenylphosphin und 110 ml THF werden bei Raumtemperatur 4,4 g Diethylazodicarboxylat zugetropft. Nach 1 h wird das Reaktionsgemisch an Rotationsverdampfer zum Rückstand eingeengt, in THF gelöst und mit 1 ml H₂O₂ versetzt. Anschließend wird mit gesättigter FeSO₄-Lösung versetzt und mit MTB-Ether extrahiert. Nach üblicher Aufarbeitung und Kugelrohrdestillation erhält man 2-Fluorethyl-4-(3,4-difluorphenyl)-phenylether, m.p. 41° C.
Analog werden hergestellt (X = Y = F, Z = H, E = -O-):

| Q | n |
|---|---|
| CH₂F | 2 |
| CH₂F | 3 |
| CH₂F | 4 |
| CH₂F | 5 |
| CF₂=CH- | 2 |
| CF₂=CH- | 3 |
| CF₂=CH- | 4 |
| CHF=CH- | 2 |
| CHF=CH- | 3 |
| CHF=CH- | 4 |

## Patentansprüche

1. Halogenierte Biphenyle der Formel I 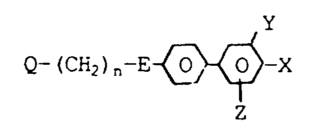 worin
Q Vinyl, CHF=CH-, CF₂=CH-, Cyclopropyl, CH₂Hal, CHHal₂ oder CHal₃,
n 1 bis 7,
E -O- oder eine Einfachbindung,
X F, Cl, -CF₃, -CN, -OCF₃ oder -OCHF₂ und
Y und Z jeweils unabhängig voneinander H oder F bedeuten.

2. Biphenyle nach Anspruch 1, dadurch gekennzeichnet, daß X = CN, Y = F und Z = H.

3. Biphenyle nach Anspruch 1, dadurch gekennzeichnet, daß X = F, Cl, -CF₃, -OCHF₂ oder -OCF₃.

4. Biphenyle nach Anspruch 3, dadurch gekennzeichnet, daß Y = Z = H.

5. Biphenyle nach Anspruch 3, dadurch gekennzeichnet, daß Y = F und Z = H oder F.

6. Verwendung der Biphenyle der Formel I nach Anspruch 1 als Komponenten flüssigkristalliner Medien für elektrooptische Anzeigen vom Supertwisttyp(STN) mit Verdrillungswinkeln von mehr als 220°.

7. Flüssigkristallines Medium für elektrooptische Anzeigen mit mindestens zwei flüssigkristallinen Komponenten, dadurch gekennzeichnet, daß mindestens eine Komponente ein Biphenyl der Formel I nach Anspruch 1 ist.

8. Elektrooptische Anzeige vom Supertwisttyp (STN) mit einem Verdrillungswinkel von mehr als 220° auf der Basis einer Flüssigkristallzelle, dadurch gekennzeichnet, daß die Flüssigkristallzelle ein Medium nach Anspruch 7 enthält.

## Claims

1. Halogenated biphenyls of the formula I 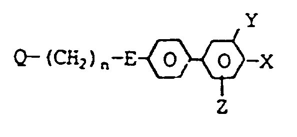 in which
Q is vinyl, CHF=CH-, CF₂=CH-, cyclopropyl, CH₂Hal, CHHal₂ or CHal₃,
n is 1 to 7,
E is -O- or a single bond,
X is F, Cl, -CF₃, -CN, -OCF₃ or -OCHF₂ and
Y and Z, each independently of the other, is H or F.

2. Biphenyls according to Claim 1, characterized in that X = CN, Y = F and Z = H.

3. Biphenyls, according to Claim 1, characterized in that X = F, Cl, -CF₃, -OCHF₂ or -OCF₃.

4. Biphenyls according to Claim 3, characterized in that Y = Z = H.

5. Biphenyls according to Claim 3, characterized in that Y = F and Z = H or F.

6. Use of the biphenyls of the formula I according to Claim 1 as components of liquid-crystalline media for electro-optical displays of the supertwist type (STN) having twist angles of greater than 220°.

7. Liquid-crystalline medium for electro-optical displays having at least two liquid-crystalline components, characterized in that at least one component is a biphenyl of the formula I according to Claim 1.

8. Electro-optical display of the supertwist type (STN) having a twist angle of greater than 220° based on a liquid-crystal cell, characterized in that the liquid-crystal cell contains a medium according to Claim 7.

## Revendications

1. Biphényles halogénés de formule I 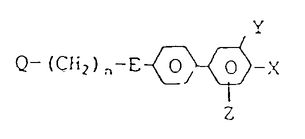 dans laquelle
Q représente un groupe vinyle, CHF=CH-, CF₂=CH-, cyclopropyle, CH₂Hal, CHHal₂ ou CHal₃,
n va de 1 à 7,
E représente -O- ou une simple liaison,
X représente F, Cl, -CF₃, -CN, -OCF₃ ou -OCHF₂, et
Y et Z représentent chacun, indépendamment l'un de l'autre, H ou F.

2. Biphényles selon la revendication 1, caractérisés en ce que X = CN, Y = F et Z = H.

3. Biphényles selon la revendication 1, caractérisés en ce que X = F, Cl, -CF₃, -OCHF₂ ou -OCF₃.

4. Biphényles selon la revendication 3, caractérisés en ce que Y = Z = H.

5. Biphényles selon la revendication 3, caractérisés en ce que Y = F et Z = H ou F.

6. Utilisation des biphényles de formule I selon la revendication 1, en tant que composants de milieux à cristaux liquides pour afficheurs électro-optiques du type superenroulé (STN) à angles de torsion de plus de 220°.

7. Milieu à cristaux liquides pour afficheurs électro-optiques à au moins deux composants de type cristal liquide, caractérisé en ce que au moins un composant est un biphényle de formule I selon la revendication 1.

8. Afficheur électro-optique du type superenroulé (STN), ayant un angle de torsion de plus de 220°, à base d'une cellule à cristaux liquides, caractérisé en ce que la cellule à cristaux liquides contient un milieu selon la revendication 7.
